# EUROPEAN PATENT SPECIFICATION

(11) **EP 4 034 188 B1**
(45) Date of publication and mention of the grant of the patent: **21.05.2025**
(21) Application number: 20867878.9
(22) Date of filing: 11.09.2020
(51) Int. Cl.: A61M 1/16, G16H 20/40, G16H 10/60, G06F 21/32, A61M 1/14, G16H 40/20, G16H 40/67, H04L 9/40, H04L 67/12, H04W 12/06, H04W 12/08, H04W 12/63

(54) **BIOMETRIC SECURITY FOR SECURE ACCESS TO A DIALYSIS MACHINE**
BIOMETRISCHE SICHERHEIT FÜR SICHEREN ZUGANG ZU EINER DIALYSEMASCHINE
SÉCURITÉ BIOMÉTRIQUE POUR UN ACCÈS SÉCURISÉ À UNE MACHINE DE DIALYSE

(30) Priority: 27.09.2019 US 201916585956
(43) Date of publication of application: 03.08.2022
(73) Proprietor: Fresenius Medical Care Holdings, Inc., Waltham, MA 02451-1457 (US)
(72) Inventor: MERICS, Thomas, Waltham, MA 02451 (US); WANG, Fei, Waltham, MA 02451 (US); RODRIGUEZ, Fred, Waltham, MA 02451 (US)
(74) Representative: Fish & Richardson P.C.
(86) International application number: PCT/US2020/050365
(87) International publication number: WO 2021/061424

(56) References cited:
- EP-A1- 1 575 004
- JP-A- 2018 015 054
- US-A1- 2004 133 787
- US-A1- 2011 307 518
- US-A1- 2014 232 525
- US-A1- 2019 258 842
- US-A1- 2019 258 842

## Description

### TECHNICAL FIELD

This patent relates generally to systems and methods for secure access to a dialysis machine.

### BACKGROUND

Medical devices, such as dialysis machines, are known for use in the treatment of renal disease. The two principal dialysis methods are hemodialysis (HD) and peritoneal dialysis (PD). During hemodialysis, the patient's blood is passed through a dialyzer of a dialysis machine while also passing dialysate through the dialyzer. A semi-permeable membrane in the dialyzer separates the blood from the dialysate within the dialyzer and allows diffusion and osmosis exchanges to take place between the dialysate and the blood stream. During peritoneal dialysis, the patient's peritoneal cavity is periodically infused with dialysate, or dialysis solution. The membranous lining of the patient's peritoneum acts as a natural semi-permeable membrane that allows diffusion and osmosis exchanges to take place between the solution and the blood stream. Automated peritoneal dialysis machines, also called PD cyclers, are designed to control the entire peritoneal dialysis process so that it can be performed at home, usually overnight, without clinical staff in attendance. Both HD and PD machines may include displays with touch screens or other user interfaces that display information of a dialysis treatment and/or enable an operator or patient to interact with the machine.

The Food and Drug Administration (FDA) recognizes that as medical devices become more digitally interconnected and interoperable, they can improve the care patients receive and create efficiencies in the health care system, but that medical devices, like computer systems, can be vulnerable to security breaches, potentially impacting the safety and effectiveness of the device. The FDA has identified and accepted a role in medical device cybersecurity that includes issuing cybersecurity guidance documents, including guidance concerning controlling secure access to the medical device to prevent unauthorized use.

It would be desirable to provide a system and techniques that address the issues noted above concerning prevention of unauthorized access to and use of a dialysis machine medical device.

US 2019/0258842 describes a device that allows for patient identification comprising a housing, a biometric sensor, coupled to the housing. the biometric sensor is configured to detect a biometric feature of a patient. The device further comprises a signal transmitter configured to transmit data related to the detected biometric feature to a medical treatment machine for carrying out a medical treatment on the patient. The patient identification device is configured to be secured to the patient during the dialysis treatment.

US 2011/0307518 describes medical record management using fingerprint identification. Fingerprints of patients, medical professionals, health payer or pharmacy personnel and potential customers are submitted to a central server and are compared with stored fingerprints of registered users. If a match is found, the user is granted an access level of authority based on the stored personal information and a "need-to-know" basis in the medical field.

### SUMMARY

Claim 1 relates to a method of securing access to a dialysis machine. Claim 9 relates to a dialysis system that uses the claimed method.

### BRIEF DESCRIPTION OF THE DRAWINGS

Embodiments and features of the system described herein are explained with reference to the several figures of the drawings, which are briefly described as follows.
FIG. 1 illustrates an exemplary embodiment of a dialysis machine in a dialysis system configured in accordance with the system described herein.
FIG. 2 shows an example of a secure access card that is configured to communicate with the communication module of the dialysis machine.
FIG. 3 is a side perspective view of the dialysis system in which the secure access card is shown being brought into close proximity to a housing of the dialysis machine.
FIG. 4 is a schematic illustration of components and processing of a biometric security system using a secure access card according to an implementation of the system described herein.
FIG. 5 is a schematic illustration of an implementation of the dialysis system with biometric security using a connected health system.
FIG. 6 is a schematic illustration of another implementation of the dialysis system with biometric security implemented using a connected health system with medical record retrieval and/or prescription download functionality.
FIG. 7 is a flow diagram showing a process for biometric proximity authentication according to one or more implementations of the system described herein. which access granted to the requesting user includes enabling the dialysis machine to obtain medical records of the patient. In an implementation, the dialysis machine receives a download over a network of a prescription of the patient for a treatment to be performed by the dialysis machine.

### BRIEF DESCRIPTION OF THE DRAWINGS

Embodiments and features of the system described herein are explained with reference to the several figures of the drawings, which are briefly described as follows.

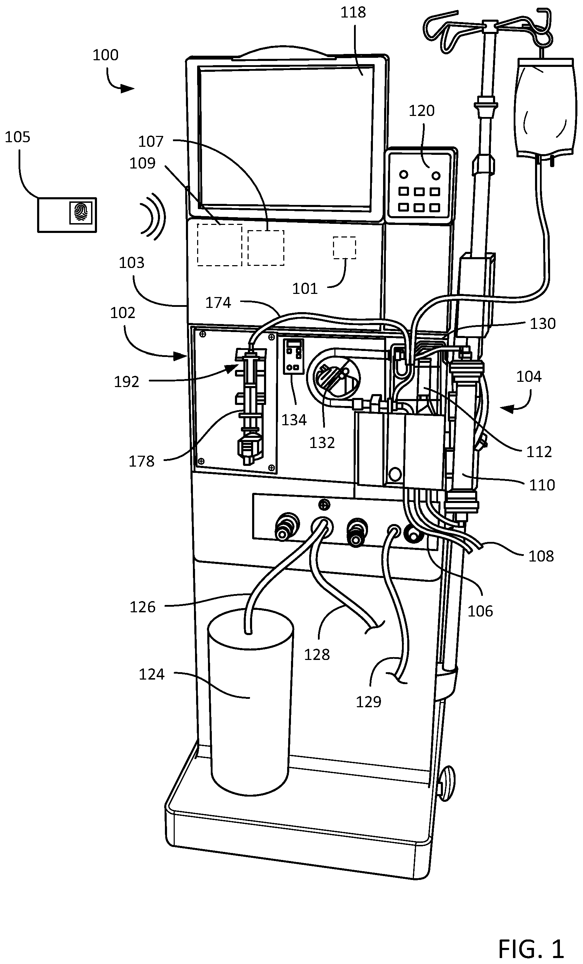

FIG. 1 illustrates an exemplary embodiment of a dialysis machine in a dialysis system configured in accordance with the system described herein.

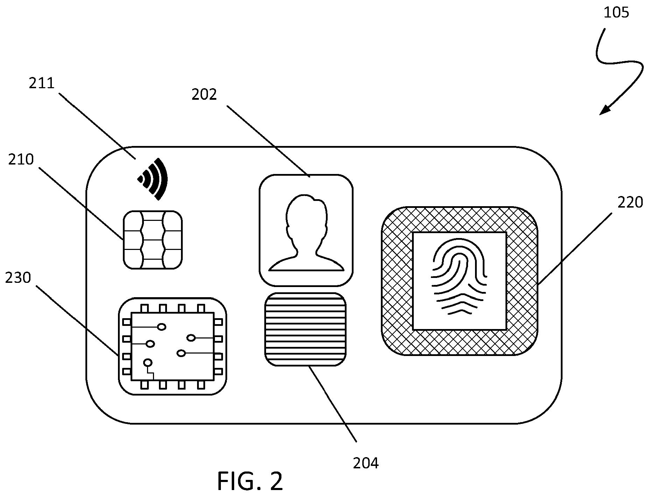

FIG. 2 shows an example of a secure access card that is configured to communicate with the communication module of the dialysis machine.

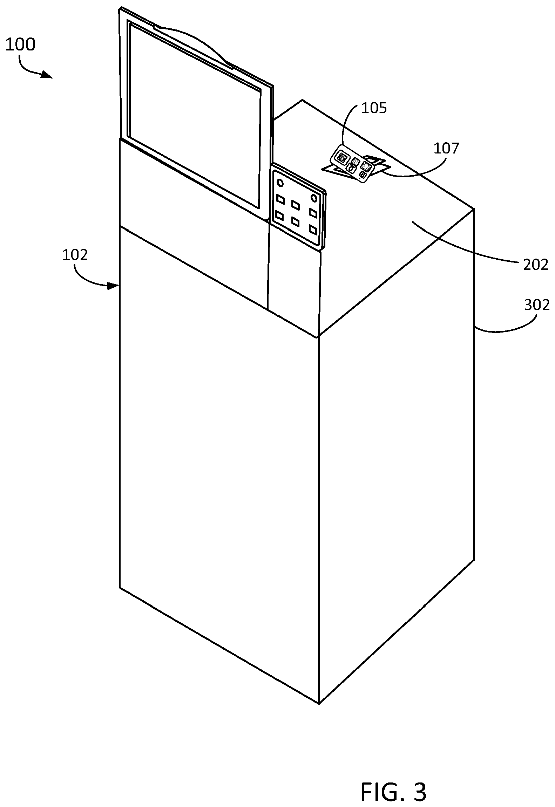

FIG. 3 is a side perspective view of the dialysis system in which the secure access card is shown being brought into close proximity to a housing of the dialysis machine.

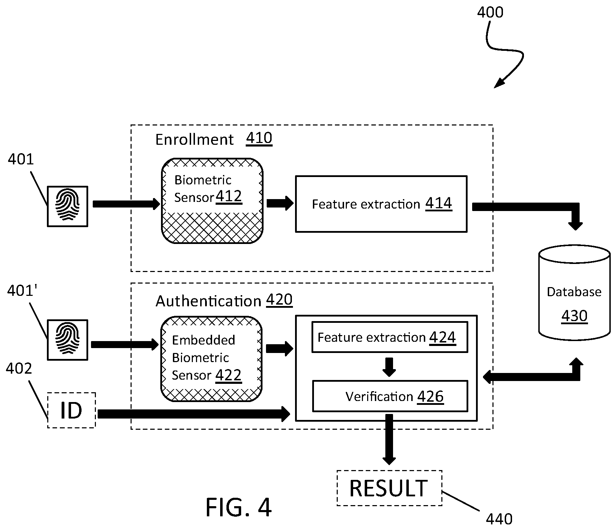

FIG. 4 is a schematic illustration of components and processing of a biometric security system using a secure access card according to an implementation of the system described herein.

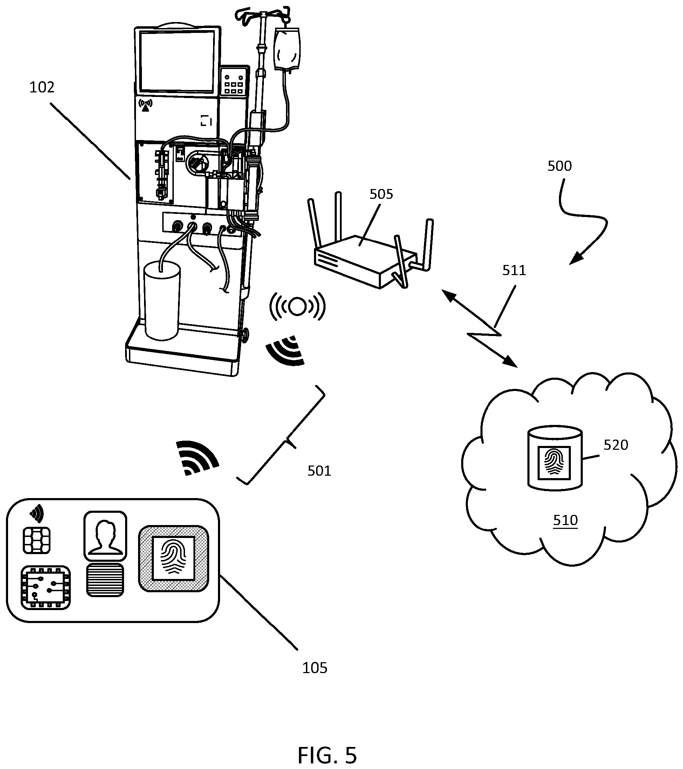

FIG. 5 is a schematic illustration of an implementation of the dialysis system with biometric security using a connected health system.

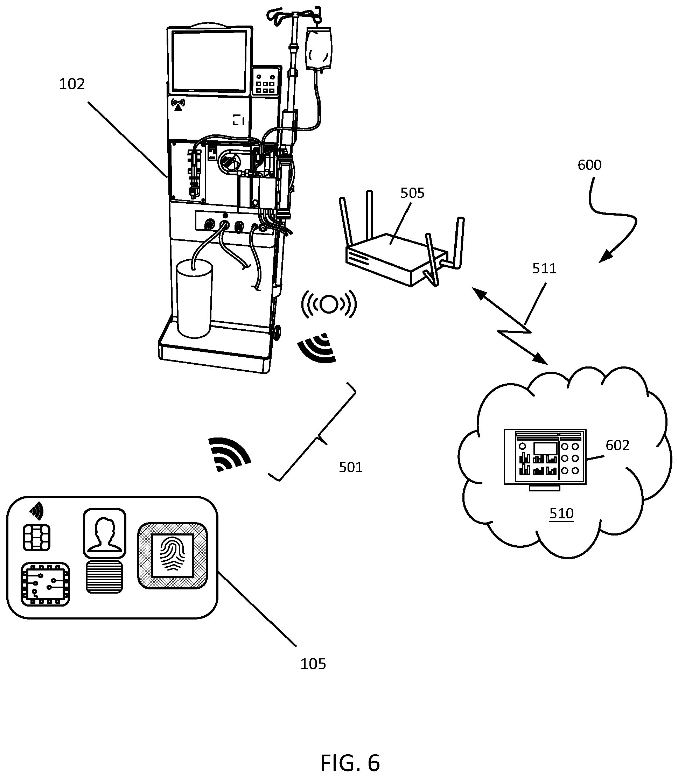

FIG. 6 is a schematic illustration of another implementation of the dialysis system with biometric security implemented using a connected health system with medical record retrieval and/or prescription download functionality.

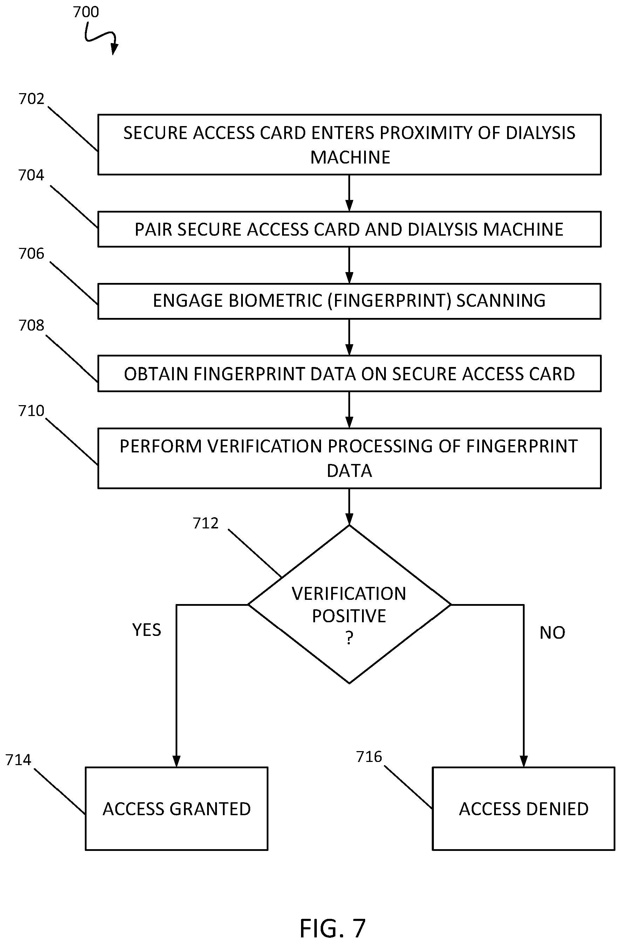

FIG. 7 is a flow diagram showing a process for biometric proximity authentication according to one or more implementations of the system described herein.

### DETAILED DESCRIPTION

Medical devices (e.g., dialysis machines) and related components (e.g. access cards, accessories and/or peripheral devices, etc.) can be configured to wirelessly communicate with each other and other devices through a connection between the devices. A connection established between devices as described herein refers to electronic communication between two or more devices such that data can be communicated between the devices. The connection may be established or a network that may include both small scale networks (e.g. a home network) and/or larger scale networks (e.g. using mobile telecommunications). The connection can be a unidirectional connection (in which data travels one way) or a bidirectional connection (in which data travels both ways). Although the present disclosure is discussed herein principally in connection with a particular type of dialysis machine, namely a hemodialysis machine, the system described herein may be used and implemented in connection with other configurations of dialysis machines, including different types of hemodialysis machines and peritoneal dialysis machines, as well as other types of medical devices for which secure access is desirably controlled.

A dialysis system may include a dialysis machine (e.g., a hemodialysis machine or a peritoneal dialysis machine) that is configured to communicate with a portable device, such as a secure access card having onboard computer processing capabilities, using a wireless connection established according to a wireless communication protocol. Implementations of the wireless connection may include a short range wireless technology protocol, such as, for example, Near Field Communication (NFC), WiFi and/or Bluetooth technology protocols. For a discussion of establishing wireless connections between medical devices, including use of short range-wireless technology protocols, reference is made to US Patent No. 9,800,663 B2 to Arrizza, entitled "Associating Dialysis Accessories Using Near Field Communication," and US Patent App. Pub. No. 2017/0087290 A1 to Medina et al., entitled "Short-Range Wireless Communication for a Dialysis System,".

According to the system described herein, the secure access card may include biometric security using biometric authentication to control secure access to the dialysis machine. Biometric authentication is the automated use of behavioral and/or physiological characteristics to verify a person's identity. An example of biometric authentication, and that is primarily discussed herein, is fingerprint authentication. The example of a secure access card will be used throughout this description, although it is noted that the system described herein may be implemented in connection with other types of smartcards, smart devices and/or access devices having onboard computer processing capabilities. Secure access cards, in particular, offer the advantages of secure access devices that are readily portable and issuable to particular patients/cardholders in connection with controlling access to specific dialysis machines and prescribed dialysis treatments. The system described herein may be used in connection with dialysis systems located in a home (e.g. home dialysis machines) and/or with dialysis systems used in a clinic or hospital environment. As discussed herein, the secure access card may control access to a dialysis machine for a cardholder, who may be, for example, a patient, clinician and/or a machine service technician.

FIG. 1 shows a dialysis system 100 configured to wirelessly communicate with a short-range wireless device, such as a secure access card 105. As further discussed in detail herein, the secure access card 105 may include biometric authentication technology that includes biometric sensing, scanning and/or other biometric processing performed on/by the secure access card 105. In an implementation, the biometric authentication may be fingerprint authentication. The dialysis system 100 may include a dialysis machine 102, e.g. a hemodialysis machine. In the hemodialysis machine implementation, as illustrated, the dialysis machine 102 is connected to a disposable blood component set 104 that partially forms a blood circuit. It is noted that the system described herein may be implemented in connection with other types of dialysis machines or medical devices, including peritoneal dialysis machines. During a hemodialysis treatment, an operator connects arterial and venous patient lines 106, 108 of the blood component set 104 to a patient. The blood component set 104 includes an air release device 112, which contains a self-sealing vent assembly that allows air but does not allow liquid to pass. As a result, if blood passing through the blood circuit during treatment contains air, the air release device 112 will vent the air to atmosphere.

The blood component set 104 is secured to a module 130 attached to the front of the dialysis machine 102. The module 130 includes the blood pump 132 capable of circulating blood through the blood circuit. The module 130 also includes various other instruments capable of monitoring the blood flowing through the blood circuit. The module 130 includes a door that when closed, as shown in FIG. 1, cooperates with the front face of the module 130 to form a compartment that is sized and shaped to receive the blood component set 104. In the closed position, the door presses certain blood components of the blood component set 104 against corresponding instruments exposed on the front face of the module 130.

The operator uses a blood pump module 134 to operate the blood pump 132. The blood pump module 134 includes a display window, a start/stop key, an up key, a down key, a level adjust key, and an arterial pressure port. The display window displays the blood flow rate setting during blood pump operation. The start/stop key starts and stops the blood pump 132. The up and down keys increase and decrease the speed of the blood pump 132. The level adjust key raises a level of fluid in an arterial drip chamber.

The dialysis machine 102 further includes a dialysate circuit formed by the dialyzer 110, various other dialysate components, and dialysate lines connected to the dialysis machine 102. Many of these dialysate components and dialysate lines are inside the housing 103 of the dialysis machine 102 and are thus not visible in FIG. 1. During treatment, while the blood pump 132 circulates blood through the blood circuit, dialysate pumps (not shown) circulate dialysate through the dialysate circuit.

A dialysate container 124 is connected to the dialysis machine 102 via a dialysate supply line 126. A drain line 128 and an ultrafiltration line 129 also extend from the dialysis machine 102. The dialysate supply line 126, the drain line 128, and the ultrafiltration line 129 are fluidly connected to the various dialysate components and dialysate lines inside the housing 103 of the dialysis machine 102 that form part of the dialysate circuit. During hemodialysis, the dialysate supply line 126 carries fresh dialysate from the dialysate container 124 to the portion of the dialysate circuit located inside the dialysis machine 102. As noted above, the fresh dialysate is circulated through various dialysate lines and dialysate components, including the dialyzer 110, that form the dialysate circuit. As will be described below, as the dialysate passes through the dialyzer 110, it collects toxins from the patient's blood. The resulting spent dialysate is carried from the dialysate circuit to a drain via the drain line 128. When ultrafiltration is performed during treatment, a combination of spent dialysate (described below) and excess fluid drawn from the patient is carried to the drain via the ultrafiltration line 129.

The dialyzer 110 serves as a filter for the patient's blood. The dialysate passes through the dialyzer 110 along with the blood, as described above. A semi-permeable structure (e.g., a semi-permeable membrane and/or semi-permeable microtubes) within the dialyzer 110 separates blood and dialysate passing through the dialyzer 110. This arrangement allows the dialysate to collect toxins from the patient's blood. The filtered blood exiting the dialyzer 110 is returned to the patient. The dialysate exiting the dialyzer 110 includes toxins removed from the blood and is commonly referred to as "spent dialysate." The spent dialysate is routed from the dialyzer 110 to a drain.

A drug pump 192 also extends from the front of the dialysis machine 102. The drug pump 192 is a syringe pump that includes a clamping mechanism configured to retain a syringe 178 of the blood component set 104. The drug pump 192 also includes a stepper motor configured to move the plunger of the syringe 178 along the axis of the syringe 178. A shaft of the stepper motor is secured to the plunger in a manner such that when the stepper motor is operated in a first direction, the shaft forces the plunger into the syringe, and when operated in a second direction, the shaft pulls the plunger out of the syringe 178. The drug pump 192 can thus be used to inject a liquid drug (e.g., heparin) from the syringe 178 into the blood circuit via a drug delivery line 174 during use, or to draw liquid from the blood circuit into the syringe 178 via the drug delivery line 174 during use.

The dialysis machine 102 includes a user interface with input devices such as a touch screen 118 and a control panel 120. The touch screen 118 and the control panel 120 allow the operator to input various different treatment parameters to the dialysis machine 102 and to otherwise control the dialysis machine 102. The touch screen 118 displays information to the operator of the dialysis system 100. The touch screen 118 can also indicate whether the secure access card 105 is in within communication range of the dialysis machine 102.

The dialysis machine 102 also includes a control unit 101 (e.g., a processor) configured to receive signals from and transmit signals to the touch screen 118, the control panel 120, and a communication module 107 (e.g., a short range wireless communication transceiver). The control unit 101 can control the operating parameters of the dialysis machine 102, for example, based at least in part on the signals received by the touch screen 118, the control panel 120, and the communication module 107. The communication module 107 is configured to communicate with a short-range wireless device using a short-range wireless technology protocol. For example, the communication module 107 allows the dialysis machine 102 to communicate with the secure access card 105.

The control unit 101 is configured to identify presence of the secure access card 105. For example, when the secure access card 105 is within wireless communication range of the communication module 107, the communication module 107 can send a signal to the control unit 101 indicating that the secure access card 105 is present. **In** response, the control unit 101 can cause the dialysis machine 102 to perform an action, as described in more detail below. Similarly, when the secure access card 105 is taken out of wireless communication range of the communication module 107 (e.g., the secure access card 105 goes from being in wireless communication range of the communication module 107 to not being in wireless communication range of the communication module 107), the communication module 107 can send a signal to the control unit 101 indicating that the secure access card 105 is not present. In response, the control unit 101 can cause the dialysis machine 102 to perform an action.

In some implementations, the dialysis system 100 may communicate via another network device, such as a gateway device, that is located in proximity to the dialysis machine (e.g. in the home) and connected via the short-range communication network to the dialysis machine 102 and that controls access to an unsecure network, such as the Internet. Data may be exchanged between the dialysis machine and a remote network or cloud-based service via a connected health system. For an example implementation of a connected health system and network that may be utilized in connection with the system described herein, reference is made to US Patent App. Pub No. 2018/0316505 A1 to Cohen et al., entitled "Securely Distributing Medical Prescriptions,".

Additionally and/or alternatively, the dialysis machine 102 may include a network communication module 109. The network communication module 109 allows the dialysis system 100 to communicate with remote servers, computer systems, databases and/or other medical devices over a network such as a local area network (LAN) or the Internet. The network communication module 109 allows the dialysis system 100 to communicate with other medical devices, computer systems, servers, and/or databases associated with one or more medical facilities. The network communication module 109 may enable communication over the network using wired and/or wireless connections. For example, the network communication module 109 may enable communication using WiFi communication protocols and infrastructure and/or may enable communication using wireless mobile telecommunication networks. The system described herein may use appropriate encryption and security standards and protocols in connection with the transmission of sensitive and/or protected data in accordance with all statutory and regulatory requirements.

FIG. 2 shows an example of an implementation of the secure access card 105 that provides biometric secure access, and/or in connection with controlled proximity access, to the dialysis machine. The secure access card 105 includes a communication module 210 (e.g., a short range wireless communication transceiver) that is configured to communicate with other communication modules using a short-range wireless technology protocol, such as the communication module 107 of the dialysis machine 102. The secure access card 105 may also include a visual indication 211 on the card concerning the wireless communication capability of the secure access card 105. In various implementations, the secure access card 105 may also include a photo 202 of the person associated with the secure access card and identification information 204 in text form. In this example, the secure access card 105 is associated with a patient, and the identification information 204 may include, for example, the patient's name, the patient's address, and a patient identification number. As further discussed elsewhere herein, in other implementations, the secure access card 105 may be associated with other types of cardholders, such as, for example, a clinician and/or a service technician.

The secure access card 105 includes a biometric authentication sensor that, in an implementation, is illustrated and described as a fingerprint sensor 220. The fingerprint sensor 220 is capable of capturing a digital image of a fingerprint pattern for a finger applied to the fingerprint sensor 220. In an implementation, the fingerprint sensor 220 may be a capacitive fingerprint sensor, although other types of fingerprint sensors may also be used, such as optical and/or thermal fingerprint sensors. In an implementation, an onboard processor module 230 on the secure access card 105 may provide the processing for the fingerprint sensor 220 and may also include a memory for storing data, such as a digital template of the user's fingerprint that is captured and stored at the time of enrollment and association of the secure access card 105 to the user. The processor module 230 thereby includes the circuitry and/or processing capability needed for the fingerprint authentication. In an implementation, the on-board processor module 230 may include a battery and/or other power supply that provides power for the module and/or other elements of the secure access card 205. Alternatively, the secure access card 105 may be powered by induction via a magnetic field and/or RF field of the dialysis machine or other corresponding peripheral component, as further discussed elsewhere herein, such that the secure access card may not require an on-board battery and/or other power supply.

Accordingly, in an implementation, the hardware of the fingerprint sensor 220 and/or the algorithm for matching and verifying the fingerprint via the process module 230 are embedded onto the secure access card 105 itself. For a discussion of example fingerprint sensing technologies and implementation of fingerprint sensor technology on access cards and devices, reference is made to "Biometric Payment Cards," Secure Technology Alliance (New Jersey, US), White paper v1.0, March 2019, and to "Biometric Technologies," Fingerprints (Fingerprint Cards AB) (Gothenburg, SE), White paper, Jan 2017.

It is noted that other types of biometric authentication sensors that may implemented on a smartcard or device, may also be used with the system described herein.

The communication modules 107, 210 may include short-range communication antennas and modules. For example, the communication modules 107, 210 may implement NFC communication as the short range communication and may be referred to as NFC initiators and NFC targets. NFC is a short-range wireless technology protocol that enables devices to establish radio communication amongst each other (e.g. paired to each other) in order to quickly exchange data over a low latency link (e.g., a link which has relatively low delay between transmission and receipt of a portion of data such as a data packet or frame). Some implementations of NFC techniques are based on standards defined by the International Electrotechnical Commission and/or the International Organization for Standardization (ISO), for example, standards such as ISO 13157 and ISO 18092. As further discussed elsewhere herein, it is noted that short range communication technologies and protocols other than NFC may be used in connection with the system described herein, including, for example, WiFi and Bluetooth communication protocols.

In some examples, the communication module 107 of the dialysis machine 102 may be an NFC initiator, and the communication module 210 of the secure access card 105 may be an NFC target. For example, the secure access card 105 may include a short-range communication technique, such as a contactless chip. Techniques for using contactless chips that could be used with the secure access card 105 may be defined, see e.g., ISO 14443. The NFC initiator can generate an RF field that powers the NFC target when the NFC target is within operable range of the NFC initiator, thereby allowing the NFC target to provide data to the NFC initiator. In this way, the secure access card 105 can provide information to the dialysis system 100.

The operable range of the NFC initiator and NFC target may be in the order of inches (e.g., 0-6 inches i.e. 0-0,1524m).

In some implementations, the transfer of data is initiated upon the NFC initiator and the NFC target making physical contact with each other. In some implementations, the NFC initiator and/or the NFC target can include a motion sensor (e.g., an accelerometer) to assist in identifying the occurrence of physical contact between the modules. It is noted that in other short-range communication protocol implementations, such as WiFi and/or Bluetooth, the operable range may be larger (e.g. 10 feet or more).

The NFC initiator is sometimes part of another electronic device such as a mobile phone, a computer, or as in this example, a medical device. The NFC initiator can have an independent power source or it can receive power from a power source that provides power to the electronic device. The NFC initiator can include a loop antenna that uses magnetic induction to generate an RF field.

The NFC target (sometimes referred to as an NFC tag) is typically a passive module that relies on the power generated by the RF field to operate. The NFC target can include a memory that stores data to be provided to the NFC initiator. The NFC target can also include a loop antenna that is configured to modulate the RF field generated by the NFC initiator. The modulation is based at least in part on the stored data. The NFC initiator can identify characteristics of the modulated field, compare them to characteristics of the generated RF field, and use the comparison information to determine the data stored on the NFC initiator. Because an implementation of the NFC target does not necessarily require its own power supply, in some implementations, the NFC target can take relatively simple form factors that can easily be incorporated into small portable devices, such as the secure access card 105. However, in other implementations, the NFC target may be powered by its own power supply. In some examples, the NFC target can also generate an RF field, and the NFC initiator can modulate the RF field generated by the NFC target in a manner similar to that described above in order to provide data to the NFC target.

The NFC initiator and NFC target can transfer data at various speeds and according to various codings. For example, data can be transferred at speeds in the range of 100-500 kbit/s according to a delay encoding scheme or a phase encoding scheme. The NFC target and/or the NFC initiator can employ an amplitude modulation scheme (e.g., an amplitude-shift keying scheme) or a phase modulation scheme (e.g., a phase-shift keying scheme), among others, to modulate the generated RF field in order to convey information.

FIG. 3 is a side perspective view of the dialysis system 100 in which the secure access card 105 is shown being brought into close proximity to a housing of the dialysis machine 102. The communication module 107 can be positioned an appropriate position on the dialysis machine 102 such that the secure access card 105 is within wireless communication range of the communication module 107 when the secure access card 105 is placed on a surface 302 (e.g., a top surface) of the housing 103 of the dialysis machine 102. For example, the communication module 107 may be positioned at or near the top of the dialysis machine 102, such as within the housing 103 at a location substantially adjacent to the surface 302. In some implementations, the surface 302 includes a recess (not shown) in which the secure access card 105 can rest such that the secure access card 105 does not easily slide off of the surface with incidental contact.

The dialysis system 100 also includes a data storage configured to store data corresponding to the more short-range wireless devices, including the secure access card 105, and the authentication processes related thereto. The data storage can be included as part of the dialysis machine 102 or may be remote from the dialysis machine 102 (e.g., on a server accessible by a computer network).

The secure access card 105 is configured to provide information related to the patient's identity to the dialysis system 100 when the secure access card 105 is in proximity to (e.g., within wireless communication range of) the communication module 107 and/or after successful biometric authorization from the results of the fingerprint sensor 220 and processing by the processing module 230. In some implementations, the biometric authorization functionality and processing of the secure access card 105 may only occur after the secure access card 105 has been brought into proximity to the communication module 107. In this way, the system described herein may require proximity of the secure access card 105 to the dialysis machine 102 and biometric authentication, e.g. fingerprint authentication, by an authorized person, thereby ensuring a requirement of biometric proximity as a security mechanism for access and/or controlling the dialysis system 100.

In connection with the biometric authentication, the dialysis system 100 can determine the patient's identity based on the received identification information communicated from the secure access card 105. In an implementation, the patient's identity is determined using the results of the biometric (fingerprint) scan performed on the secure access card 105. Moreover, the dialysis system 100 may access the data storage that stores data corresponding to the secure access card 105, and use identified information concerning the patient's name and/or ID (or, e.g., the corresponding value) received from the secure access card 105 to identify records of the patient. In response, the dialysis machine 102 can perform an action that is based at least in part on the identity of the patient.

In some implementations, the data corresponding to the one or more secure access cards can include portions of patient records, such as each associated patient's name, address, phone number, identification number, and the like. The data corresponding to identities of one or more short-range wireless devices can include data representing the value that corresponds to the patient's name and/or ID number. The dialysis system 100 can query the data corresponding to identities of one or more short-range wireless devices using the patient name, ID, and/or value received from the secure access card 105 to find the corresponding patient record and determine the identity of the patient. In various implementations, the data may be stored on the dialysis system 100 and periodically updated, such as via transmission using storage devices, e.g., having universal serial bus (USB) interfaces, that are transferred between the dialysis system 100 and a remote computer and/or site. In other implementations, the data may be obtained by the dialysis system 100 using real-time communication over a network, as described in further detail elsewhere herein.

The data corresponding to the secure access card 105 can also be used to access information such as each associated patient's medical history, treatment prescriptions, treatment parameters, and the like. Examples of treatment parameters include a dialysate type, a dialysate fill volume, and a dialysate flow rate, to name a few. Upon determining that the secure access card 105 belongs to a particular patient, the dialysis system 100 can request and download, for example, using a connected health system as further discussed elsewhere herein, prescription treatment information corresponding to a particular treatment for that patient and cause the dialysis machine 102 to carry out that treatment. The control unit 101 can cause the dialyzer 110 to carry out the dialysis treatment based on the downloaded prescription.

For example, suppose that the patient associated with the secure access card 105, John Doe, has a medical condition that requires an atypical dialysis treatment. Perhaps John's treatment requires an abnormally low dialysate flow rate. The secure access card 105 is used to biometrically authenticate the user John Doe when the secure access card 105 is in proximity to the dialysis system 100, as further discussed elsewhere herein. After biometric proximity authentication, the dialysis system 100 receives the patient identification information from the secure access card 105, accesses remote data storage, and uses the received patient identification information to identify medical information related to John Doe. The medical information includes John Doe's medical history, treatment prescriptions, and treatment parameters; in particular, the treatment prescription includes instructions for causing the dialysis machine 102 to employ the abnormally low dialysate flow rate that John Doe requires. Such information is obtained (e.g. downloaded) and provided to the control unit 101, and the control unit 101 causes the appropriate treatment to be administered to John Doe. For example, the control unit 101 can cause the dialyzer 110 to operate a pump (e.g., a dialysate pump) such that the required dialysate flow rate is achieved.

FIG. 4 is a schematic illustration of components and processing of a biometric security system 400 using a secure access card according to an implementation of the system described herein. The system 400 includes an enrollment module 410 and a biometric authentication module 420. The enrollment module 410 is where a biometric template of an authorized user 401 is initially obtained, for example, using a biometric sensor 412, such as a fingerprint sensor, that scans a fingerprint of the authorized user 401 at a time of enrollment. In various implementations, the biometric sensor 412 may be incorporated on a tablet computer, a stand-alone fingerprint sensor, and/or as part of a machine at an enrollment location. In another implementation, the biometric sensor 412 may be the sensor embedded on the secure access card 105 when the secure access card 105 is brought into proximity of a site or computer that performs enrollment processing. A feature extraction module 414 extracts features from the scanned biometric data, e.g. extracted fingerprint feature data, and stores them in a database 430. In various implementations, as further discussed elsewhere herein, the database 430 may be a remote database as part of a connected health system, a database stored on a medical device, such as the dialysis machine 102, and/or a database that is accessible directly on or by the secure access card 102.

After enrollment processing of the authorized user 401 by the enrollment module 410, an access-requesting user 401' may request access to a medical device, such as the dialysis machine 102, by presenting the secure access card 102, which access request requires biometric acquisition and verification by the authentication module 420 according to an implementation of the system described herein. The authentication by the authentication module 420 may be initiated when the requesting user 401' brings the secure access card 102 into proximity of the dialysis machine 102. At that time, the secure access card 102 may pair with the dialysis machine 102, and information, such as ID information 402, associated with the authorized user 401 of the secure access card 102 may be transmitted. An embedded biometric sensor 422 of the secure access card 102, such as the fingerprint sensor 220, may be activated and a biometric/fingerprint scan of the user 401' acquired using the embedded biometric sensor 422. Identifying features of the biometric data scanned by the sensor 422 are extracted at a feature extraction module 424 that may, for example, be performed on the secure access card 105. Using the ID information 402, the biometric template data for the authorized user 401 that has been previously stored is obtained from the database 430. The stored biometric data is matched to the scanned/acquired biometric data in a verification module 426. In various implementations, the verification module 426 may be located/processed on the secure access card 105, at the dialysis machine 102, and/or at a remote site, and a result 440, e.g. either a positive or negative match, is determined. The result 440 may be used to determine whether access to the dialysis machine 102 by the requesting user 401' is granted or denied.

FIG. 5 is a schematic illustration of an implementation of the dialysis system 100 with biometric security implemented using a connected health system 500. In an implementation, as discussed in further detail below, the biometric template information (e.g. fingerprint matching template) may be stored on a server or database 520 accessible via a secure network connection 511 that may be established over an unsecure and/or cloud-based network 510, such as via the Internet, using the connected health system. Enrollment of the cardholder, and acquisition of the enrolled biometric (fingerprint) data, may occur elsewhere and stored in the database 520 for access in response to a biometric authentication request.

As illustrated, the connected health system 500 may include components that enable establishment of a secure network connection 511 with a remote server, database or cloud-based service, see, e.g., database 520 shown in the network 510. In an implementation, as illustrated, the secure network connection 511 may be established using a gateway device 505 that controls secure access to the unsecure network, such as the Internet, and in some implementations, may establish connection using a wide area network and/or a mobile telecommunications network. The gateway device 505 may then further control establish of a short-range network connection 401, such as an NFC, WiFi or Bluetooth network connection that enables short range communication among components of the dialysis system 100, such as the gateway device 505, the dialysis machine 102 and the secure access card 105 (and/or any other peripheral devices of the dialysis system 100, such as a connected blood pressure cuff and/or a connected weight scale, for example). In an implementation, the dialysis system 100 and other short-range networked connected components may be implemented in a patient's home for a home dialysis treatment.

In an implementation, for biometric proximity authentication according to the system described herein, the secure access card 105 is brought into proximity of the dialysis machine 102, such as via an NFC connection, as further discussed elsewhere herein. The cardholder desiring access to the dialysis machine 102 then invokes the biometric authentication, for example, by applying a finger to the fingerprint sensor 220 on the secure access card 105. The fingerprint sensor 220 scans the fingerprint of the cardholder and, in the illustrated embodiment, sends data corresponding to the scanned fingerprint to the dialysis machine 102, e.g. via wireless short-range transmission. It is noted that, in an embodiment, the fingerprint data may be sent using the via the short-range communication network 501 (e.g. NFC, WiFi and/or Bluetooth) to the dialysis machine 102. The short-range communication network 501 may be established using wireless components of the dialysis machine 102 (e.g. module 107) and/or using the gateway device 505 shown in the figure. Thereafter, in an implementation, the obtained fingerprint data may be sent via the secure network communication 511 via the cloud-based network 510 to the database 520 for matching verification. The fingerprint matching verification is performed remotely and a result of that matching verification, either positive or negative, is returned to the dialysis machine 102. If verified, the then cardholder is thereby authenticated as permitted to access the dialysis machine 102.

Alternatively, in another implementation, using ID and/or other identification information obtained from the secure access card concerning the enrolled cardholder, corresponding fingerprint data may be retrieved by the gateway device 505 and/or the dialysis machine 101 over the secure network communication 411 from the database 520 via the cloud-based network 510. The fingerprint matching verification may be then be performed at the gateway device 505 and/or at the dialysis machine 102.

FIG. 6 is a schematic illustration of another implementation of the dialysis system 100 with biometric security implemented using a connected health system 600 with medical record retrieval and/or prescription download functionality. In some implementations, data corresponding to a patient/cardholder enrolled to the secure access card 105 is stored remote from the dialysis system 100. For example, the data can be stored on a computer system, server, and/or database 602 that is associated with a medical facility corresponding to that of the dialysis system 100, e.g., accessible via the cloud-based network 510. The computer system, server, and/or database 602 may be a medical database in which patient information is stored. In this way, the dialysis system 100 can receive portions of patient records, including prescriptions for treatment, from a remote location, e.g. obtained via communication over a network, when the secure access card 105 is within wireless communication range of the communication module 107 and biometric authentication has occurred via the secure access card 105. The dialysis system 100 can then use the received information to identify the patient and determine the patient's medical history, treatment prescriptions, treatment parameters, and the like. In an implementation, after biometric proximity authentication using the secure access card 105 according to the system described herein, a current prescription of the patient/cardholder may be downloaded using the connected health system 600 and delivered to the dialysis machine 102 in connection with performance of a dialysis treatment for the patient/cardholder.

FIG. 7 is a flow diagram 700 showing processing for biometric authentication using a secure access card (e.g. secure access cards 105) according to one or more implementations of the system described herein. At a step 702, the secure access card 105 is brought into proximity of a dialysis machine (e.g. dialysis machine 102). The secure access card 105 is issued to and carried by a patient/cardholder and used to provide biometric authentication for providing secure access when the secure access card is in proximity to the dialysis machine 102. At a step 704, the secure access card 105 and the dialysis machine 102 are paired to register that the secure access card 105 has been brought into proximity of the dialysis machine 102, for example, via a short-range wireless protocol, such as NFC. At a step 706, the patient/cardholder engages the biometric sensing or scanning of the secure access card 105, such as applying a finger to the fingerprint sensor embedded on the secure access card 105. At a step 708, the fingerprint sensor of the secure access cards 105 obtains the biometric fingerprint data of the patient/cardholder.

At a step 710, the fingerprint data undergoes verification processing by matching the obtained fingerprint data to stored fingerprint template data of the patient/cardholder that has been previously obtained, e.g. at a time of enrollment of the patient/cardholder with the secure access card 105. The fingerprint matching according to the system described herein may occur in different locations according to various implementations. For example, in an implementation, the fingerprint matching verification may occur on the secure access card 105 using an embedded algorithm of the onboard processing of the secure access cards 105. The result of the fingerprint verification may then be communicated to the dialysis machine 102. In other implementations, the fingerprint data obtained from the fingerprint scan may be transmitted form the secure access card to the dialysis machine 102. The dialysis machine may then either transmit the fingerprint data via a network to a remote computer and/or database, e.g. via a connected health system, for fingerprint verification matching and receive thereafter the result from the remote computer and/or database. Alternatively, the dialysis machine 102 may send a request to the remote computer and/or database for transmission to the dialysis machine 102 of the previously stored fingerprint template data patient/cardholder enrolled to the secure access cards 105. Thereafter, the fingerprint verification matching may occur at the dialysis machine 102 (and/or via a locally connected component of the dialysis machine, such as a gateway device).

At a decision step 712, the result of the fingerprint matching is analyzed to assess if the verification is a positive match. If the verification is positively matched (YES), meaning the obtained fingerprint data matches the enrolled template fingerprint data, then at a step 714, the patient/cardholder who presented the secure access card 105 at the dialysis machine 102 is granted access to the dialysis machine 102, and which may include the obtaining and/or downloading of records corresponding to the patient/cardholder. If the verification is not positively matched (NO) at the decision step 712, then at a step 716, access to the dialysis machine 102 is denied. In an embodiment, the access may be access by a patient and/or caregiver and included access to the patient's medical records and treatment prescription. In another embodiment, the access may be access by a service technician to service the dialysis machine.

Implementations discussed herein may be combined with each other in appropriate combinations in connection with the system described herein. Additionally, in some instances, the order of steps in the flow diagrams, flowcharts and/or described flow processing may be modified, where appropriate. The system may further include a display and/or other computer components for providing a suitable interface with a user and/or with other computers. Aspects of the system described herein may be implemented or controlled using software, hardware, a combination of software and hardware and/or other computer-implemented or computer-controlled modules or devices having described features and performing described functions. Data exchange and/or signal transmissions to, from and between components of the system may be performed using wired or wireless communication. This communication may include use of one or more transmitter or receiver components that securely exchange information via a network, such as an intranet or the Internet, and may include use of components of local area networks (LANs) or other smaller scale networks, such as Wi-Fi, Bluetooth or other short range transmission protocols, and/or may include use of components of wide area networks (WANs) or other larger scale networks, such as mobile telecommunication networks.

Software implementations of aspects of the system described herein may include executable code that is stored in a computer-readable medium and executed by one or more processors. The computer-readable medium may include volatile memory and/or non-volatile memory, and may include, for example, a computer hard drive, ROM, RAM, flash memory, portable computer storage media, an SD card, a flash drive or other drive with, for example, a universal serial bus (USB) interface, and/or any other appropriate tangible or non-transitory computer-readable medium or computer memory on which executable code may be stored and executed by a processor. The system described herein may be used in connection with any appropriate operating system. The meanings of any method steps of the invention(s) described herein are intended to include any suitable method of causing one or more parties or entities to perform the steps unless a different meaning is expressly provided or otherwise clear from the context.

As used herein, an element or operation recited in the singular and preceded with the word "a" or "an" should be understood as not excluding plural elements or operations, unless such exclusion is explicitly recited. References to "one" embodiment or implementation of the present disclosure are not intended to be interpreted as excluding the existence of additional embodiments that also incorporate the recited features. Furthermore, a description or recitation in the general form of "at least one of [a], [b] or [c]," or equivalent thereof, should be generally construed to include [a] alone, [b] alone, [c] alone, or any combination of [a], [b] and [c].

Implementations of the invention will be apparent to those skilled in the art from a consideration of the specification or practice of the invention disclosed herein. It is intended that the specification and examples be considered as exemplary only, with the true scope of the invention being indicated by the following claims.

## Claims

1. A method of securing access to a dialysis machine (102), the method comprising:
controlling access, via a secure access card (105), to the dialysis machine (102), wherein the secure access card includes a biometric security module embedded on the secure access card, and wherein the biometric security module includes a biometric sensor (220) that obtains biometric data from a requesting user of the secure access card; and
performing verification processing, via a biometric verification module, to verify the obtained biometric data from the requesting user in relation to template biometric data previously stored during an enrollment of an authorized user that associates the authorized user to the secure access card, wherein either:
a) the user is a service technician and the access granted to the requesting user includes access allowing the requesting user to service the dialysis machine, or
b) the requesting user is a caregiver of the patient and the access granted to the requesting user includes enabling the dialysis machine to obtain medical records of the patient.

2. The method according to claim 1, wherein the biometric sensor is a fingerprint sensor.

3. The method according to claim 1 or claim 2, wherein the secure access card further comprises a first short-range wireless transceiver (107) that pairs with a second short-range wireless transceiver (210) on the dialysis machine to enable transmission of signals between the first and second short-range wireless transceivers when the secure access card is in proximity to the dialysis machine.

4. The method according to any of claims 1-3, further comprising:
activating the biometric sensor of the secure access card only after the secure access card is determined to be in proximity to the dialysis machine.

5. The method according to any of claims 1-4, wherein the biometric security module embedded on the secure access card includes the biometric sensor that obtains the biometric data from the requesting user and the biometric verification module that performs processing to verify the biometric data obtained from the requesting user.

6. The method according to any of claims 1-5, wherein the biometric verification module is located remotely from the secure access card.

7. The method according to any of claims 1-6, wherein, after the biometric validation module verifies the biometric data in relation to the template biometric data of the authorized user, access to the dialysis machine is granted to the requesting user.

8. The method according to any of claims 1-7, further comprising:
downloading to the dialysis machine over a network a prescription of the patient for a treatment to be performed by the dialysis machine.

9. A dialysis system comprising:
a dialysis machine;
a secure access card (105) that controls access to the dialysis machine, wherein the secure access card includes a biometric security module embedded on the secure access card, and wherein the biometric security module includes a biometric sensor configured to obtain biometric data from a requesting user of the secure access card; and
a biometric verification module that performs processing to verify the obtained biometric data from the requesting user in relation to template biometric data previously stored during an enrollment of an authorized user that associates the authorized user to the secure access card,
wherein the dialysis system is configured to perform the method of any one of claims 1 to 8.

10. The dialysis system according to claim 9, wherein the biometric sensor of the secure access card is activated only after the secure access card is determined to be in proximity to the dialysis machine.

11. The dialysis system according to any of the preceding claims 9-10, wherein the biometric security module embedded on the secure access card includes the biometric sensor that obtains the biometric data from the requesting user and the biometric verification module that performs processing to verify the biometric data obtained from the requesting user.

12. The dialysis system according to any of the preceding claims 9-11, wherein, after the biometric validation module verifies the biometric data in relation to the template biometric data of the authorized user, access to the dialysis machine is granted to the requesting user.

13. The dialysis system according to any of the preceding claims 9-12, wherein the dialysis machine is configured to receive a download over a network of a prescription of the patient for a treatment to be performed by the dialysis machine.

14. The dialysis system according to any of the preceding claims 9-13, wherein the dialysis machine (102) comprises
a user interface with input devices (118, 120) configured to allow an operator to input various different treatment parameters to the dialysis machine (102) and to otherwise control the dialysis machine (102); and
a control unit (101) configured to receive signals from and transmit signals to the input devices (118, 120) and a communication module (107), wherein the control unit (101) is configured to control operating parameters of the dialysis machine (102), and the communication module (107) is configured to communicate with a short-range wireless secure access card using a short-range wireless technology protocol.

## Patentansprüche

1. Verfahren zur Sicherung des Zugriffs auf eine Dialysemaschine (102), wobei das Verfahren Folgendes umfasst:
Steuern von Zugriff auf die Dialysemaschine (102) über eine sichere Zugriffskarte (105), wobei die sichere Zugriffskarte ein biometrisches Sicherheitsmodul aufweist, das in die sichere Zugriffskarte eingebettet ist, und wobei das biometrische Sicherheitsmodul einen biometrischen Sensor (220) aufweist, der biometrische Daten von einem anfordernden Benutzer der sicheren Zugriffskarte erhält; und
Durchführen einer Verifizierungsverarbeitung über ein biometrisches Verifizierungsmodul, um die erhaltenen biometrischen Daten von dem anfordernden Benutzer in Bezug auf biometrische Vorlagendaten zu verifizieren, die zuvor während einer Registrierung eines autorisierten Benutzers gespeichert wurden und die den autorisierten Benutzer mit der sicheren Zugriffskarte assoziieren, wobei entweder
a) der Benutzer ein Wartungstechniker ist und der dem anfordernden Benutzer gewährte Zugriff einen Zugriff aufweist, der es dem anfordernden Benutzer erlaubt, die Dialysemaschine zu warten, oder
b) der anfordernde Benutzer ein Betreuer des Patienten ist und der dem anfordernden Benutzer gewährte Zugriff ein Ermöglichen aufweist, dass die Dialysemaschine medizinische Datensätze des Patienten erhält.

2. Verfahren nach Anspruch 1, wobei der biometrische Sensor ein Fingerabdrucksensor ist.

3. Verfahren nach Anspruch 1 oder 2, wobei die sichere Zugriffskarte einen ersten drahtlosen Nahbereichs-Sendeempfänger (107) umfasst, der mit einem zweiten drahtlosen Nahbereichs-Sendeempfänger (210) auf der Dialysemaschine gekoppelt wird, um Übertragung von Signalen zwischen dem ersten und dem zweiten drahtlosen Nahbereichs-Sendeempfänger zu ermöglichen, wenn die sichere Zugriffskarte in der Nähe der Dialysemaschine ist.

4. Verfahren nach einem der Ansprüche 1-3, ferner umfassend:
Aktivieren des biometrischen Sensors der sicheren Zugriffskarte nach dem Bestimmen, dass die sichere Zugriffskarte in der Nähe der Dialysemaschine ist.

5. Verfahren nach einem der Ansprüche 1-4, wobei das biometrische Sicherheitsmodul, das in die sichere Zugriffskarte eingebettet ist, den biometrischen Sensor, der die biometrischen Daten von dem anfordernden Benutzer erhält, und das biometrische Verifizierungsmodul aufweist, das Verarbeitung zum Verifizieren der biometrischen Daten durchführt, die von dem anfordernden Benutzer erhalten werden.

6. Verfahren nach einem der Ansprüche 1-5, wobei das biometrische Verifizierungsmodul entfernt von der sicheren Zugriffskarte angeordnet ist.

7. Verfahren nach einem der Ansprüche 1-6, wobei nach dem Verifizieren der biometrischen Daten in Bezug auf die biometrischen Vorlagendaten des autorisierten Benutzers durch das biometrische Validierungsmodul dem anfordernden Benutzer Zugriff auf die Dialysemaschine gewährt wird.

8. Verfahren nach einem der Ansprüche 1-7, ferner umfassend:
Herunterladen einer Patientenverschreibung für eine durch die Dialysemaschine durchzuführende Behandlung über ein Netzwerk auf die Dialysemaschine.

9. Dialysesystem umfassend:
eine Dialysemaschine;
eine sichere Zugriffskarte (105), die den Zugriff auf die Dialysemaschine steuert, wobei die sichere Zugriffskarte ein biometrisches Sicherheitsmodul aufweist, das in die sichere Zugriffskarte eingebettet ist, und wobei das biometrische Sicherheitsmodul einen biometrischen Sensor aufweist, der dazu ausgelegt ist, biometrische Daten von einem anfordernden Benutzer der sicheren Zugriffskarte zu erhalten; und
ein biometrisches Verifizierungsmodul, das Verarbeitung zum Verifizieren der erhaltenen biometrischen Daten von dem anfordernden Benutzer in Bezug auf biometrische Vorlagendaten durchführt, die zuvor während einer Registrierung eines autorisierten Benutzers gespeichert wurden und die den autorisierten Benutzer mit der sicheren Zugriffskarte assoziieren,
wobei das Dialysesystem dazu konfiguriert ist, das Verfahren nach einem der Ansprüche 1 bis 8 durchzuführen.

10. Dialysesystem nach Anspruch 9, wobei der biometrische Sensor der sicheren Zugriffskarte nur nach dem Bestimmen aktiviert wird, dass die sichere Zugriffskarte in der Nähe der Dialysemaschine ist.

11. Dialysesystem nach einem der vorhergehenden Ansprüche 9-10, wobei das biometrische Sicherheitsmodul, das in die sichere Zugriffskarte eingebettet ist, den biometrischen Sensor, der die biometrischen Daten von dem anfordernden Benutzer erhält, und das biometrische Verifizierungsmodul aufweist, das Verarbeitung zum Verifizieren der biometrischen Daten durchführt, die von dem anfordernden Benutzer erhalten werden.

12. Dialysesystem nach einem der vorhergehenden Ansprüche 9-11, wobei nach dem Verifizieren der biometrischen Daten in Bezug auf die biometrischen Vorlagendaten des autorisierten Benutzers durch das biometrische Validierungsmodul dem anfordernden Benutzer Zugriff auf die Dialysemaschine gewährt wird.

13. Dialysesystem nach einem der vorhergehenden Ansprüche 9-12, wobei die Dialysemaschine dazu ausgelegt ist, über ein Netzwerk einen Download einer Patientenverschreibung für eine durch die Dialysemaschine durchzuführende Behandlung zu empfangen.

14. Dialysesystem nach einem der vorhergehenden Ansprüche 9-13, wobei die Dialysemaschine (102) Folgendes umfasst:
eine Benutzerschnittstelle mit Eingabevorrichtungen (118, 120), die dazu ausgelegt sind, einem Bediener zu ermöglichen, verschiedene Behandlungsparameter in die Dialysemaschine (102) einzugeben und die Dialysemaschine (102) anderweitig zu steuern; und
eine Steuereinheit (101), die dazu ausgelegt ist, Signale von den Eingabevorrichtungen (118, 120) und einem Kommunikationsmodul (107) zu empfangen und Signale an diese zu senden, wobei die Steuereinheit (101) dazu ausgelegt ist, Betriebsparameter der Dialysemaschine (102) zu steuern, und das Kommunikationsmodul (107) dazu ausgelegt ist, unter Verwendung eines Protokolls für die drahtlose Nahbereichstechnologie mit einer sicheren drahtlosen Nachbereichskarte zu kommunizieren.

## Revendications

1. Procédé de sécurisation de l'accès à une machine de dialyse (102), le procédé comprenant :
la commande de l'accès, par le biais d'une carte d'accès sécurisé (105), à la machine de dialyse (102), dans lequel la carte d'accès sécurisé comprend un module de sécurité biométrique intégré dans la carte d'accès sécurisé, et dans lequel le module de sécurité biométrique comporte un capteur biométrique (220) qui obtient des données biométriques d'un utilisateur demandeur de la carte d'accès sécurisé ; et
la réalisation d'un traitement de vérification, par le biais d'un module de vérification biométrique, pour vérifier les données biométriques obtenues de l'utilisateur demandeur par rapport à des données biométriques types précédemment stockées lors d'un enrôlement d'un utilisateur autorisé qui associe l'utilisateur autorisé à la carte d'accès sécurisé, dans lequel soit :
a) l'utilisateur est un technicien d'entretien et l'accès accordé à l'utilisateur demandeur comporte un accès permettant à l'utilisateur demandeur d'entretenir la machine de dialyse, soit
b) l'utilisateur demandeur est un soignant du patient et l'accès accordé à l'utilisateur demandeur comporte l'activation de la machine de dialyse pour obtenir des dossiers médicaux du patient.

2. Procédé selon la revendication 1, dans lequel le capteur biométrique est un capteur d'empreintes digitales.

3. Procédé selon la revendication 1 ou la revendication 2, dans lequel la carte d'accès sécurisé comprend en outre un premier émetteur-récepteur sans fil à courte portée (107) qui s'appaire à un second émetteur-récepteur sans fil à courte portée (210) sur la machine de dialyse pour permettre la transmission de signaux entre les premier et second émetteurs-récepteurs sans fil à courte portée lorsque la carte d'accès sécurisé se trouve à proximité de la machine de dialyse.

4. Procédé selon l'une quelconque des revendications 1 à 3, comprenant en outre :
l'activation du capteur biométrique de la carte d'accès sécurisé uniquement après avoir déterminé que la carte d'accès sécurisé se trouve à proximité de la machine de dialyse.

5. Procédé selon l'une quelconque des revendications 1 à 4, dans lequel le module de sécurité biométrique intégré dans la carte d'accès sécurisé comporte le capteur biométrique qui obtient les données biométriques de l'utilisateur demandeur et le module de vérification biométrique qui effectue un traitement pour vérifier les données biométriques obtenues de l'utilisateur demandeur.

6. Procédé selon l'une quelconque des revendications 1 à 5, dans lequel le module de vérification biométrique est situé à distance de la carte d'accès sécurisé.

7. Procédé selon l'une quelconque des revendications 1 à 6, dans lequel, après que le module de validation biométrique a vérifié les données biométriques par rapport aux données biométriques types de l'utilisateur autorisé, l'accès à la machine de dialyse est accordé à l'utilisateur demandeur.

8. Procédé selon l'une quelconque des revendications 1 à 7, comprenant en outre :
le téléchargement, vers la machine de dialyse sur un réseau, d'une ordonnance du patient pour un traitement à réaliser par la machine de dialyse.

9. Système de dialyse comprenant :
une machine de dialyse ;
une carte d'accès sécurisé (105) qui commande l'accès à la machine de dialyse, dans lequel la carte d'accès sécurisé comporte un module de sécurité biométrique intégré dans la carte d'accès sécurisé, et dans lequel le module de sécurité biométrique comporte un capteur biométrique configuré pour obtenir des données biométriques d'un utilisateur demandeur de la carte d'accès sécurisé ; et
un module de vérification biométrique qui effectue un traitement pour vérifier les données biométriques obtenues de l'utilisateur demandeur par rapport à des données biométriques types précédemment stockées lors de l'enrôlement d'un utilisateur autorisé qui associe l'utilisateur autorisé à la carte d'accès sécurisé,
dans lequel le système de dialyse est configuré pour réaliser le procédé selon l'une quelconque des revendications 1 à 8.

10. Système de dialyse selon la revendication 9, dans lequel le capteur biométrique de la carte d'accès sécurisé est activé uniquement après avoir déterminé que la carte d'accès sécurisé se trouve à proximité de la machine de dialyse.

11. Système de dialyse selon l'une quelconque des revendications 9 à 10 précédentes, dans lequel le module de sécurité biométrique intégré dans la carte d'accès sécurisé comporte le capteur biométrique qui obtient les données biométriques de l'utilisateur demandeur et le module de vérification biométrique qui réalise un traitement pour vérifier les données biométriques obtenues de l'utilisateur demandeur.

12. Système de dialyse selon l'une quelconque des revendications 9 à 11 précédentes, dans lequel, après que le module de validation biométrique a vérifié les données biométriques par rapport aux données biométriques types de l'utilisateur autorisé, l'accès à la machine de dialyse est accordé à l'utilisateur demandeur.

13. Système de dialyse selon l'une quelconque des revendications 9 à 12 précédentes, dans lequel la machine de dialyse est configurée pour recevoir un téléchargement sur un réseau d'une ordonnance du patient pour un traitement à réaliser par la machine de dialyse.

14. Système de dialyse selon l'une quelconque des revendications 9 à 13 précédentes, dans lequel la machine de dialyse (102) comprend
une interface utilisateur avec des dispositifs d'entrée (118, 120) configurés pour permettre à un opérateur de saisir différents paramètres de traitement divers dans la machine de dialyse (102) et autrement de commander la machine de dialyse (102) ; et
une unité de commande (101) configurée pour recevoir des signaux des dispositifs d'entrée (118, 120) et transmettre des signaux à ceux-ci, et un module de communication (107), dans lequel l'unité de commande (101) est configurée pour commander les paramètres de fonctionnement de la machine de dialyse (102), et le module de communication (107) est configuré pour communiquer avec une carte d'accès sécurisé sans fil à courte portée à l'aide d'un protocole de technologie sans fil à courte portée.
